# EUROPÄISCHE PATENTSCHRIFT

(11) **EP 1 385 578 B1**
(45) Veröffentlichungstag und Bekanntmachung des Hinweises auf die Patenterteilung: **16.08.2006**
(21) Anmeldenummer: 02730250.4
(22) Anmeldetag: 10.05.2002
(51) Int. Cl.: A61P 27/16, A61P 9/00, A61K 31/70, A61K 36/00

(54) **VERWENDUNG VON RUTINEN UND AESCINEN ZUR BEHANDLUNG AURIKULÄRER DURCHBLUTUNGSSTÖRUNGEN**
UTILIZATION OF RUTINS AND AESCINS IN THE TREATMENT OF CIRCULATORY DISTURBANCES OF THE EAR
UTILISATION DE RUTINES ET D'AESCINES POUR TRAITER DES TROUBLES DE LA CIRCULATION SANGUINE DES OREILLES

(30) Priorität: 10.05.2001 DE 10122715
(43) Veröffentlichungstag der Anmeldung: 04.02.2004
(73) Patentinhaber: Brench AG, 80802 München (DE)
(72) Erfinder: GAURI, Kailash, Kumar, 24576 Hitzhusen (DE)
(74) Vertreter: Kinzebach, Werner
(86) Internationale Anmeldenummer: PCT/EP2002/005177
(87) Internationale Veröffentlichungsnummer: WO 2002/089918

(56) Entgegenhaltungen:
- EP-A- 0 900 563
- WO-A-98/51291
- DE-A- 3 402 259
- FR-A- 2 181 454
- US-A- 4 983 626
- DATABASE WPI Week 197850 Derwent Publications Ltd., London, GB; AN 1978-004257 XP002217172 & RO 64 207 A (INTR ANTIBIOTICE), 20. März 1978 (1978-03-20) & RO 64 207 A (INTR ANTIBIOTICE) 20. März 1978 (1978-03-20)

## Beschreibung

Die vorliegende Erfindung betrifft die Verwendung von Rutinen und Aescinen zur Herstellung pharmazeutischer Mittel zur Behandlung aurikulärer Durchblutungsstörungen. Beschrieben werden auch Mittel mit einer entsprechenden Wirkstoffkombination sowie Mittel in Form von Handelspackungen mit entsprechenden Kombinationspräparaten oder Monopräparaten zur kombinierten Anwendung.

Rutine sind in vielen Pflanzenarten vorkommende Glycoside des Flavons Quercetin. Das unter dem Freinamen (INN) Rutosid bekannte Rutin wird meist in Form der sauren Natriumsalze gegen kapillare Blutungen und weitere mit gesteigerter Kapillarbrüchigkeit und Membrandurchlässigkeit einhergehende Zustände eingesetzt. Es wurde daher oft als sogenannter Antipermeabilitätsfaktor oder als Vitamin P bezeichnet.

Anstatt Rutin werden vielfach auch synthetische Rutin-Derivate verwendet. Hierzu gehören insbesondere O-(β-Hydroxyethyl)-rutine, die häufig als Gemisch von 1-fach bis 4-fach und an unterschiedlichen Positionen des Quercetins mit Hydroxyethyl-Gruppen substituierten Rutinen anfallen. Ein wichtiger Vertreter dieser Derivate ist das Troxerutin, das als Hauptkomponente eines Gemisches von O-(β-Hydroxyethyl)-rutinen oder als reine Substanz zur Behandlung von Erkrankungen der Venen und Folgezuständen, insbesondere bei chronisch-venöser Insuffizienz, Varicosis, Ulcus cruris und Thrombophlebitis eingesetzt werden kann. Weitere Indikationsfelder sollen opthalmologische Anwendungen sein, wie Retinophathia diabetica, Retina- und Glaskörperblutungen, subkonjunktivale Blutungen und Thrombosen.

Auch bei Aescin, einem aus Roßkastanien isolierbaren Saponin-Gemisch, handelt es sich um ein geläufiges Venentherapeutikum, das als Aescin-haltiger Roßkastaniensamen-Extrakt oder als aufgereinigtes Asecin wegen einer ödemprotektiven bzw. antiexsudativen Wirkung geschätzt wird.

Dementsprechend werden im Bereich der Venentherapeutika auch Kombinationspräparate angeboten, die Vertreter beider Wirkstoffklassen aufgreifen. So werden beispielsweise Roßkastaniensämenextrakte mit 0-(β-Hydroxyethyl)-rutinen oder Rutin-Schwefelsäureestern bei venösen Durchblutungsstörungen, wie Ödemen, Wadenkrämpfen, Juckreiz sowie Schmerzen und Schwergefühl in den Beinen, durch Krampfadern bedingten Schwellungen und Stauungszuständen, Varicosis und postthrombotischem Syndrom, Unterschenkelgeschwüren, Hämorrhoiden, sowie posttraumatischen und postoperativen Weichteilschwellungen empfohlen (vgl. Rote Liste 2000, Aulendorf: ECV, Editio Cantor Verlag, Einträge 83044 und 83046).

Ferner nennt die WO 98/51291 unter anderem Rutosid, Troxerutin oder Aescin zur Behandlung und Prävention ischämischer Störungen.

Aurikuläre Durchblutungsstörungen führen insbesondere dann, wenn sie das Innenohr betreffen, zu einem häufig irreversiblen Funktionsverlust des Ohres, d.h. einem eingeschränkten Hörvermögen oder gar Taubheit.

Deshalb sind aurikuläre Durchblutungsstörungen ernstzunehmende Symptome, die einer effektiven Behandlung bedürfen. Sind diese Symptome auf systemische Gefäßerkrankungen zurückzuführen, so erfolgt in der Regel eine Behandlung der Grunderkrankung. Allerdings stellt der auf diese Weise erreichbare, auf die Symptomatik am Ohr gerichtete Therapieerfolg oftmals nicht zufrieden.

Deshalb ist es in diesen Fällen vielfach notwendig, neben einer Behandlung der Grunderkrankung weitere Maßnahmen zu ergreifen.

Die zu diesem Zweck auf dem Gebiet der Ohrenheilkunde in der Regel verordneten Hörgeräte sind jedoch wegen der mangelnden Akzeptanz durch den Patienten und der Verstärkung sämtlicher Geräusche von Nachteil. Auch systemische Therapien mit durchblutungsfördernden Mitteln, wie z.B. Pentoxyphyllin oder Pentyphyllin, bieten für den Bereich aurikulärer Durchblutungsstörungen keine zufriedenstellende Behandlungsmöglichkeit.

Es wurde nun gefunden, daß bestimmte kombinierte Anwendungen von Rutinen und Aescinen eine überraschend effektive Behandlungsmöglichkeit aurikulärer Durchblutungsstörungen eröffnen.

Gegenstand der vorliegenden Erfindung ist daher die Verwendung wenigstens eines Flavonoids aus der Gruppe der Rutine, nämlich von Rutin, physiologisch akzeptabler Derivate und/oder Salze davon, in Kombination mit wenigstens einem Saponin aus der Gruppe der Aescine, nämlich von Aescin, physiologisch akzeptabler Derivate und/oder Salze davon, zur Behandlung aurikulärer Durchblutungsstörungen.

Die erfindungsgemäße Verwendung von Rutin, physiologisch akzeptabler Derivate oder Salze davon - zwecks Vereinfachung auch als "Rutine" oder "Rutin-Komponente" bezeichnet - und die Verwendung von Aescin, physiologisch akzeptabler Derivate oder Salze davon - zwecks Vereinfachung auch als "Aescine" oder "Aescin-Komponente" bezeichnet - bietet wesentliche Vorteile bei der Behandlung von aurikulären Durchblutungsstörungen.

Die Rutin-Komponente und die Aescin-Komponente können prinzipiell gemeinsam in einer Formulierung oder getrennt in wenigstens zwei verschiedenen Formulierungen verabreicht werden. Die letztere Möglichkeit beinhaltet sowohl die gleichzeitige als auch die zeitlich beabstandete, d.h. zu unterschiedlichen Zeitpunkten erfolgende, Verabreichung. Eine besondere Ausführungsform der zeitlich beabstandeten Verabreichung wird durch die abwechselnde Verabreichung beider Komponenten, beispielsweise mit einem Früh/Spät-Tagesrhythmus, realisiert.

In diesem Sinne sind Gegenstand der Erfindung Mittel zur Behandlung aurikulärer Durchblutungsstörungen, die auf einer Kombination aus wenigstens einem Rutin, eines physiologisch akzeptablen Derivates und/oder Salzes davon und wenigstens einem Aescin, eines physiologisch akzeptablen Derivates und/oder Salzes davon, sowie gegebenenfalls weiteren Wirkstoffen basieren, wobei die Wirkstoffkomponenten, insbesondere die Rutin- und Aescin-Komponente, gemeinsam oder getrennt formuliert sein können.

"Rutin" bezeichnet erfindungsgemäß 3-[[6-O-(6-Deoxy-α-L-mannopyranosyl)-β-D-glucopyranosyl]oxy]-2-(3,4-dihydroxyphenyl)-5,7-dihydroxy-4H-1-benzopyran-4-on, auch Quercetin-3-rutinosid oder Rutosid (INN) genannt, der Formel I

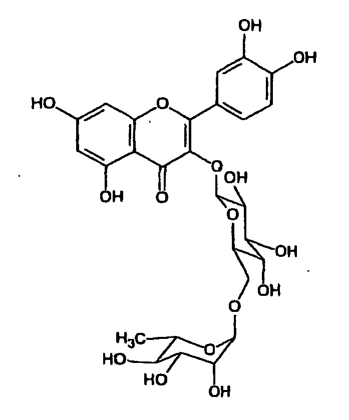

Zu den Rutin-Derivaten gehören vor allem O-(β-Hydroxyethyl)-Rutine, insbesondere die entsprechenden Mono-, Bis-, Tris- und Tetra-(Hydroxyethyl)-Derivate einschließlich der jeweiligen regioisomeren Formen. Beispielsweise sei Monoxerutin, d.h. 7-Mono-O-(β-Hydroxyethyl)-rutin und vor allem Troxerutin, d.h. 3',4',7-Tris-O-(β-Hydroxyethyl)-Rutin der Formel II 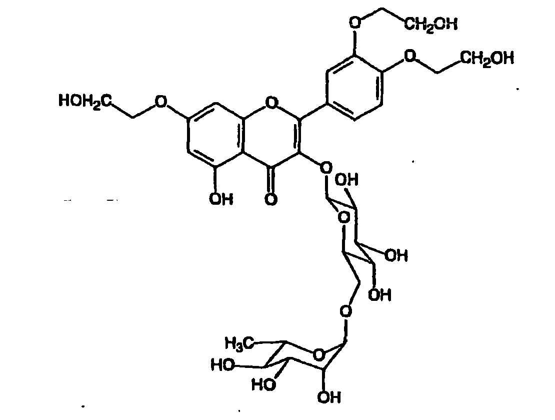 genannt.

Zu weiteren physiologisch akzeptablen Derivaten des Rutins gehören Ethoxazorutin, 8,8'-Methylenbis[6-diethylaminomethylrutin], Rutinschwefelsäureester, Diosmin (2,3-Dehydrohesperidin).

Zu den physiologisch akzeptablen Salzen von Rutin bzw. Rutin-Derivaten gehören im vorliegenden Fall bevorzugt Basenadditionssalze, die insbesondere mit sauren Estern, z.B. den Schwefelsäureestern, des Rutins gebildet werden.

Zu den Basenadditionssalzen zählen Salze mit anorganischen Basen, beispielsweise Metallhydroxiden bzw. -carbonaten von Alkali-, Erdalkali- oder Übergangsmetallen, oder mit organischen Basen, beispielsweise Ammoniak oder basischen Aminosäuren, wie Arginin und Lysin, Aminen, z.B. Methylamin, Dimethylamin, Trimethylamin, Triethylamin, Ethylamin, Diethylamin, Ethylendiamin, Ethanolamin, Diethanol-amin, 1-Amino-2-propanol, 3-Amino-1-propanol oder Hexamethylentetraamin, gesättigten cyclischen Aminen mit 4 bis 6 Ringkohlenstoffatomen, wie Piperidin, Piperazin, Pyrrolidin und Morpholin, sowie weiteren organischen Basen, beispielsweise N-Methylglucamin, Kreatin und Tromethamin, sowie quaternären Ammoniumverbindungen, wie Tetramethylammonium und dergleichen.

Bevorzugt sind Salze mit anorganischen Basen, z.B. Na-, K-, Mg-, Ca-, Zn-, Cr- und Fe-Salze.

Rutin kann aus natürlichen Quellen, insbesondere den Blütenknospen von Sophora japonica oder Buchweizenkraut gewonnen werden. Beispielsweise kann man das Drogenmaterial zunächst mit heißem Wasser oder niederen Alkoholen ausziehen, die gewonnenen Extrakte einengen und gegebenenfalls mit geeigneten Lösungsmitteln entfetten. Das sich beim Erkalten abscheidende Roh-Rutin kann dann aus Wasser oder Ethanol umkristallisiert bzw. durch Zugabe von Alkali gelöst und mit Säuren wieder ausgefällt werden.

Darüber hinaus ist Rutin auch synthetisch zugänglich, beispielsweise indem man 7,4'-Dibenzylquercetin mit Hexaacetobromrutinose unter geeigneten Bedingungen umsetzt, beispielsweise in Pyridin in Gegenwart von Ag₂CO₃ koppelt. Anschließend können die Acetylestergruppen verseift und die Benzylschutzgruppen, zum Beispiel hydrogenolytisch über Pd/C, abgespalten werden. Erforderlichenfalls wird das Roh-Rutin umkristallisiert, beispielsweise aus Methanol.

O-(β-Hydroxyethyl)-Rutine können durch Hydroxyethylierung der phenolischen Gruppen des Rutins mit geeigneten Reagenzien wie 2-Chlorethanol oder Glycochlorhydrin erhalten werden. In der Regel wird diese Reaktion im alkalischen Medium, beispielsweise in Gegenwart von NaOH geführt.

Der Begriff "Aescin" beschreibt ein aus Roßkastanien (Aesculus hippocastanum) und insbesondere aus deren Samen isolierbares Saponin-Gemisch von hauptsächlich diacetylierten Tetra- und Pentahydroxy-β-amyrin-Verbindungen, die in Position 3 eine mit Zuckerresten, zum Beispiel Glucose, Galactose und/oder Xylose, substituierte Glucuronsäure tragen. Die Aglyka sind bekannt unter der Bezeichnung Barringtogenol der Formel III 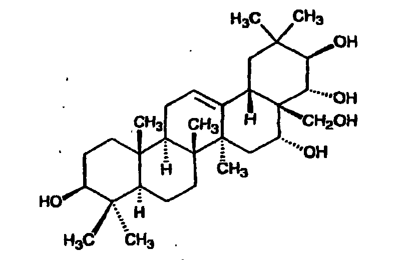 sowie Protoaescigenin der Formel IV

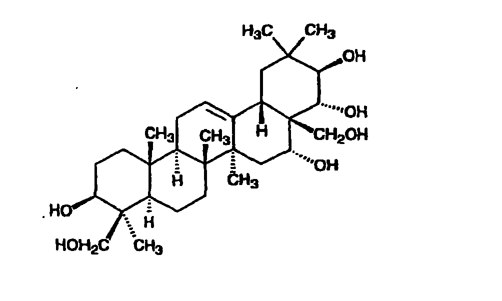

In Position 21 sind unterschiedliche Mengen Angelica-, Tiglin-, α-Methylbutter- und Isobuttersäure esterartig gebunden. Der Begriff "Aescin" umfasst α-Aescin, β-Aescin und Krypto-Aescin, welche Acetylgruppen in unterschiedlichen Positionen, beispielsweise am 22-α-Hydroxyl (β-Aescin) oder am 28-Hydroxyl (Krypto-Aescin), tragen. Vorzugsweise werden die Aescine als Roßkastaniensamenextrakt oder in isolierter Form verwendet.

Zu geeigneten Roßkastaniensamenextrakten gehören Fluidextrakte, die mit Alkohol-Wasser-Mischungen erhältlich sind, sowie Trockenextrakte, die aus den Fluidextrakten durch anschließende Trocknung, vorzugsweise Sprühtrocknung, gewonnen werden können. Als Auszugsmittel eignen sich beispielsweise wäßriges Ethanol oder Methanol. Gute Aescin-Ausbeuten werden z.B. durch Extraktion mit 40 bis 60%-igem Ethanol oder Methanol erzielt. Geläufig sind insbesondere eingestellte Trockenextrakte (4-8:1), die auf Triterpenglycoside, berechnet als Aescin, standardisiert sind.

Isoliertes Aescin kann man aus Roßkastaniensamen beispielsweise mittels Chromatographie unter Verwendung von Ionenaustauschern (Harzen) isolieren.

Zur weiteren Erläuterung sei beispielsweise auf die EP 0 900 563 A1 Bezug genommen, welche die Herstellung Aescin-haltiger pharmazeutischer Zubereitungen betrifft.

Zu den physiologisch akzeptablen Derivaten von Aescin gehören im vorliegenden Fall Ester mit organischen Säuren, nämlich Carbonsäuren, z.B. Essigsäure, Weinsäure, Milchsäure, Citronensäure, Äpfelsäure, Mandelsäure, Ascorbinsäure, Maleinsäure, Fumarsäure, Gluconsäure oder Sulfonsäuren, z. B. Methansulfonsäure, Benzolsulfonsäure und Toluolsulfonsäure, und dergleichen. Derartige Säuren sind vorwiegend an eine oder mehrere OH-Gruppen in Position 21, 22 und auch 28 gebunden. Als zweckmäßig hat sich beispielsweise das Tartrat erwiesen.

Neben den Rutin- und Aescin-Komponenten kann die erfindungsgemäße Behandlung weitere Wirkstoffe miteinbeziehen. Bei diesen Wirkstoffen kann es sich insbesondere um solche handeln, deren Wirkung der Rutin- bzw. Aescin-vermittelten Wirkung ähnlich ist oder diese ergänzt. So kann es von Vorteil sein, zusätzlich zur erfindungsgemäßen Kombination, Otologika, Venentherapeutika, Antihämorrhagika, Antikoagulantia, und ähnliche Wirkstoffe zu verabreichen. Insbesondere kann es zweckmäßig sein, antiphlogistische Wirkstoffe vom Corticoidtyp, z.B. Glucocorticoide, oder vom nicht-corticoiden Typ, wie z.B. Indometazin, oder Acetylsalicylsäure oder Derivate davon, zu verabreichen. Ebenso kann es zweckmäßig sein, thrombolytische Wirkstoffe, wie z.B. Streptokinase oder Urokinase, und Antikoagulantia wie z.B. Cumarinderivate zu verabreichen.

Eine besondere Ausführungsform der vorliegenden Erfindung basiert auf der Kombination von Troxerutin mit Aescin oder einem physiologisch akzeptablen Salz davon.

Unter aurikulären Durchblutungsstörungen versteht man Durchblutungsstörungen, die das Ohr bzw. Teile davon betreffen bzw. dessen oder deren Funktion beeinträchtigen. Hierzu zählen vor allem Durchblutungsstörungen des Innenohrs, insbesondere des Bereichs der Stria vascularis, der Cochlea, des Bereichs des Hörnerven, des Ganglion spirale oder des Hörbahnbereichs, sowie Durchblutungsstörungen des zentralen Hörzentrums im Gehirn, insbesondere des Temorallappens des Gehirns. Gemäß einer besonderen Ausführungsform betrifft die vorliegende Erfindung daher die akute oder präventive Behandlung von Durchblutungsstörungen des Innenohrs und der Hörbahn. Mitunter gehören zu den erfindungsgemäß behandelbaren Durchblutungsstörungen auch solche Störungen, die zu aurikulären Durchblutungsstörungen führen können, ohne dass zum Behandlungszeitpunkt ein solcher Zustand gegeben sein muß. Hierzu zählen beispielsweise Durchblutungsstörungen im vertebrobasiliären Stromgebiet, chronische Durchblutungsstörungen im Rahmen einer Blutdruck-Regulationstörung, oder beispielsweise eine generelle Arteriosklerose oder Angiopathie in folge einer Stoffwechselerkrankung wie z.B. Diabetes mellitus. Eine Behandlung solcher Störungen stellt eine Prävention aurikulärer Durchblutungsstörungen dar.

Zu erfindungsgemäß behandelbaren Störungen, deren Ursache aurikuläre Durchblutungsstörungen sind, gehören vor allem Erkrankungen, die auf venöse Insuffizienzen, insbesondere auf thrombotische Veränderungen der ohrversorgenden Astvenen oder Zentralvenen, und/oder arterielle Insuffizienzen, z.B. Teleangiektasien zurückzuführen sind. Zu diesen Störungen gehören vor allem Schwerhörigkeit und insbesondere Altersschwerhörigkeit (Presbyucusis) sowie Ohrgeräusche (Tinnitus aureus) und vasculär bedingter Hörsturz (sowohl einseitig als auch beidseitig) und die damit einhergenden Einschränkungen des Hörvermögens. Ferner gehören auch vestibuläre Symptome, insbesondere Gleichgewichtsstörungen und/oder Schwindel dazu.

Bevorzugte Ausführungsformen der vorliegenden Erfindung richten sich auf die Behandlung von cochlearen, insbesondere vaskulär bedingten, Hörstörungen, wozu Presbyacusis, Hypacusis, Dysacusis, Hörsturz, Tinnitus aureus, Schallempfindlichkeitsschwerhörigkeit und ferner auch vestibuläre Symptome wie Gleichgewichtstörung und/oder Schwindel zählen.

Die erfindungsgemäße Verwendung gewinnt bei Erwachsenen mit zunehmendem Lebensalter an Bedeutung. In der Gruppe der über 40-jährigen und vor allem der über 50-jährigen bringt die Behandlung besondere Vorteile mit sich. Eine weitere Gruppe, bei denen die erfindungsgemäße Behandlung besondere Vorteile mit sich bringen kann, bilden Patienten, bei denen als cochleare, vaskulär bedingte Hörstörung ein Hörsturz oder einer Gleichgewichtsstörung vorliegt.

Erfindungsgemäß zu behandelnde Erkrankungen sind häufig gekennzeichnet durch eine progressive Entwicklung, d.h. die vorstehend beschriebenen Zustände verändern sich im Laufe der Zeit, in der Regel nimmt der Schweregrad zu und gegebenenfalls können Zustände ineinander übergehen oder weitere Zustände zu bereits bestehenden Zuständen hinzutreten.

Ein besonderer Aspekt einer Behandlung im erfindungsgemäßen Sinne betrifft die Behandlung akuter oder chronischer Störungen. Die Behandlung kann symptomatisch, beispielsweise als Symptomsuppression ausgerichtet sein. Sie kann kurzzeitig erfolgen, mittelfristig ausgerichtet sein, oder es kann sich auch um eine Langzeitbehandlung, beispielsweise im Rahmen einer Erhaltungstherapie, handeln.

Erfindungsgemäß wird dem zu behandelnden Individuum, vorzugsweise einem Säuger, insbesondere einem Menschen und auch einem Nutz-oder Haustier, eine wirksame Menge Rutin-Komponente und eine wirksame Menge Aescin-Komponente, in der Regel der pharmazeutischen, tierarzneilichen oder lebensmitteltechnologischen Praxis entsprechend formuliert, verabreicht.

Die Behandlung erfolgt in der Regel durch einmaliges oder mehrmaliges tägliches Verabreichen einer geeigneten Dosis gegebenenfalls zusammen oder im Wechsel mit anderen Wirkstoffen oder wirkstoffhaltigen Präparaten, so dass einem zu behandelnden Individuum von etwa 75 kg Körpergewicht eine Tagesdosis von etwa 10 mg bis 20 g, vorzugsweise von etwa 200 mg bis 10 g, vorteilhafterweise vonvon etwa 900 mg bis 5 g, und insbesondere von etwa 2 g bis 3 g Rutin-Komponente sowie von etwa 500 µg bis 1 g, vorzugsweise von etwa 1 mg bis 500 mg und insbesondere von etwa 5 mg bis 200 mg Aescin-Komponente, bei oraler Gabe, sowie von etwa 10 mg bis 20 g Rutin-Komponente bzw. etwa 25 µg bis 500 mg Aescin-Komponente bei parenteraler oder auch intraaurikulärer Gabe verabreicht wird. Unabhängig von der Aescin-Dosis stellt die Verabreichung einer oralen Tagesdosis von mehr als 1 g, vorzugsweise von mehr als 1,8 g und insbesondere von mehr als 2 g Rutin-Komponente einen besonders vorteilhaften Aspekt der Erfindung dar.

Wirkstoffmengen und -anteile beziehen sich auf den aktiven Wirkstoff, so dass für Salze und Derivate eine entsprechende Umrechnung zu erfolgen hat. Eine Anpassung an das Körpergewicht kann erforderlich sein.

Die Erfindung betrifft auch die Herstellung von Mitteln zur Behandlung eines Individuums, vorzugsweise eines Säugers, insbesondere eines Menschen und auch eines Nutz- oder Haustieres.

Ein Aspekt der vorliegenden Erfindung sind daher auch Mittel, enthaltend
i) wenigstens ein Flavonoid aus der Gruppe der Rutine, nämlich Rutin, physiologisch akzeptable Derivate und/oder Salze davon, und
ii) wenigstens ein Saponin aus der Gruppe des Aescine, nämlich Aescin, physiologisch akzeptable Derivate und/oder Salze davon, sowie
gegebenenfalls wenigstens einen weiteren Wirkstoff und eine Formulierungsgrundlage.

Erfindungsgemäße Mittel basieren daher auf einer Wirkstoffkombination und gegebenenfalls einer Formulierungsgrundlage.

Zu den Mitteln gehören insbesondere pharmazeutische Mittel, womit auch veterinärmedizinische Mittel gemeint sind. Otologische Mittel stellen eine besondere Ausführungsform dar.

Die Wirkstoffkombination im Sinne der Erfindung umfaßt als Wirkstoffkomponente i) wenigstens ein Rutin, d.h. Rutin, physiologisch akzeptable Derivate und/oder Salze davon. Gemische dieser Formen sind möglich und in bestimmten Fällen in Betracht zu ziehen. Gemäß einer besonderen Ausführungsform besteht die Wirkstoffkomponente i) aus einem O-(β-Hydroxyethyl)rutin-Gemisch, das Troxerutin als Hauptkomponente, vorzugsweise zu mindestens 50 Gew.-% und insbesondere zu mindestens 80 Gew.-% enthält. Gemäß einer weiteren besonderen Ausführungsform besteht die Wirkstoffkomponente i) im wesentlichen aus Troxerutin. Die gewichtsprozentualen Angaben sind auf das Gesamtgewicht der Wirkstoffkomponente i) bezogen.

Die Wirkstoffkombination im Sinne der Erfindung umfaßt als Wirkstoffkomponente ii) wenigstens ein Aescin, d.h. Aescin, physiologisch akzeptable Derivate und/oder Salze davon. Gemische dieser Formen sind möglich und in bestimmten Fällen in Betracht zu ziehen. Gemäß einer besonderen Ausführungsform besteht die Wirkstoffkomponente ii) aus einem Roßkastaniensamenextrakt, der vorzugsweise etwa 10 bis 30 Gew.-% Aescin enthält. Gemäß einer weiteren besonderen Ausführungsform besteht die Wirkstoffkomponente ii) im wesentlichen aus Aescin. Die gewichtsprozentualen Angaben sind auf das Gesamtgewicht der Wirkstoffkomponente ii) bezogen.

Weiterhin kann die Wirkstoffkombination im Sinne der Erfindung als Wirkstoffkomponente iii) weitere Wirkstoffe umfassen, beispielsweise die oben in diesem Zusammenhang genannten Wirkstoffe.

Der Anteil der Wirkstoffkombination an der Formulierung ist größer als ein gegebenenfalls in natürlichen Quellen vorhandener Anteil. In diesem Sinne sind die erfindungsgemäßen Mittel im Hinblick auf die Wirkstoffkombination angereichert. Im Falle eines pharmazeutischen Mittels liegt der Anteil in der Regel bei etwa 1 bis 60 Gew.-%, vorzugsweise bei etwa 5 bis 35 Gew.-% und insbesondere bei etwa 10 bis 30 Gew.-%.

Angaben in Gew.-% beziehen sich, sofern nicht anderes angegeben ist, auf das Gesamtgewicht der Formulierung.

Die Formulierungsgrundlage erfindungsgemäßer Formulierungen enthält physiologisch akzeptable Hilfsstoffe. Physiologisch akzeptabel sind die im Bereich der Pharmazie, der Lebensmitteltechnologie und angrenzenden Gebieten bekanntermaßen verwendbaren Hilfstoffe, insbesondere die in einschlägigen Arzneibüchern (z.B. DAB, Ph. Eur., BP, NF) gelisteten, und auch andere Hilfstoffe, deren Eigenschaften einer physiologischen Anwendung nicht entgegenstehen.

Geeignete Hilfsstoffe können sein: Netzmittel; emulgierende und suspendierende Mittel; konservierende Mittel; Antioxidantien; Antireizstoffe; Chelatbildner; Dragierhilfsmittel; Emulsionsstabilisatoren; Filmbildner; Gelbildner; Geruchsmaskierungsmittel; Geschmackskorrigentien; Harze; Hydrokolloide; Lösemittel; Lösungsvermittler; Neutralisierungsmittel; Permeationsbeschleuniger; Pigmente; quaternäre Ammoniumverbindungen; Rückfettungs- und Überfettungsmittel; Salben-, Creme- oder Öl-Grundstoffe; Silikon-Derivate; Spreithilfsmittel; Stabilisatoren; Sterilanzien; Suppositoriengrundlagen; Tabletten-Hilfsstoffe, wie Bindemittel, Füllstoffe, Gleitmittel, Sprengmittel oder Überzüge; Treibmittel; Trocknungsmittel; Trübungsmittel; Verdickungsmittel; Wachse; Weichmacher; Weißöle. Eine diesbezügliche Ausgestaltung beruht auf fachmännischem Wissen, wie beispielsweise in Fiedler, H.P., Lexikon der Hilfsstoffe für Pharmazie, Kosmetik und angrenzende Gebiete, 4. Auflage, Aulendorf: ECV-Editio-Kantor-Verlag, 1996, dargestellt ist.

Die Summe aus Wirkstoffkomponente und Formulierungsgrundlage beträgt in der Regel 100 Gew.-%.

Beispiele geeigneter pharmazeutischer Formulierungen sind feste Arzneiformen, wie Pulver, Puder, Granulate, Tabletten, insbesondere Filmtabletten, Pastillen, Sachets, Cachets, Dragees, Kapseln wie Hart- und Weichgelatinekapseln, Suppositorien oder vaginale Arzneiformen, halbfeste Arzneiformen, wie Salben, Cremes, Hydrogele, Pasten oder Pflaster, sowie flüssige Arzneiformen, wie Lösungen, Emulsionen, insbesondere Öl-in-Wasser-Emulsionen, Suspensionen, beispielsweise Lotionen, Injektions- und Infusionszubereitungen, Ohrentropfen. Auch implantierte Abgabevorrichtungen können zur Verabreichung erfindungsgemäßer Wirkstoffe verwendet werden. Ferner können auch Liposomen oder Mikrosphären zur Anwendung kommen. Bevorzugt sind feste Arzneiformen und insbesondere Tabletten und Kapseln.

Die Formulierungen können beispielsweise auf oralem, rektalem, transdermalem, subkutanem, intravenösem, intramuskulärem, intraaurikulärem oder intranasalem Weg verabreicht werden. Bevorzugt ist die orale Verabreichung.

Bei der Herstellung der Zusammensetzungen werden die Wirkstoffe gewöhnlich mit einem geeigneten Hilfsstoff, in diesem Fall auch als Exzipient zu bezeichnen, vermischt oder verdünnt. Exzipienten können feste, halbfeste oder flüssige Materialien sein, die als Vehikel, Träger oder Medium für den Wirkstoff dienen. Die Zumischung weiterer Hilfsstoffe erfolgt erforderlichenfalls in an sich bekannter Weise. Es können Formgebungsschritte, gegebenenfalls in Verbindung mit Mischvörgangen, durchgeführt werden, z.B. eine Granulierung, Komprimierung und ähnliches.

Insbesondere können die Wirkstoffkomponenten gemeinsam formuliert werden. Sie können aber auch zunächst getrennt verarbeitet und anschließend in einer kompartimentierten, z.B. mehrschichtigen Arzneiform zusammengeführt werden. Dadurch kann möglichen Wirkstoffinkompatibilitäten und unterschiedlichen Wirkstoffeigenschaften, wie Bioverfügbarkeit, Stabilität, Löslichkeit und ähnlichem, Rechnung getragen werden.

Ebenso betrifft die Erfindung entsprechender Monopräparate in Form von Handelspackungen, denen die erfindungsgemäße kombinierte Anwendung zu entnehmen ist.

Die vorliegende Erfindung wird anhand der nachfolgenden Beispiele näher erläutert, ohne darauf beschränkt zu sein.

### Beispiel 1

### Pharmazeutische Mittel

### a) Weichgelatine-Kapsel mit Troxerutin und Aescin (Troxerutin 450 mg + Aescin 25 mg)

Füllung:

| | |
|---|---|
| Troxerutin | 450 mg |
| Aescin | 25 mg |
| Sojaöl (raff.) | 440 mg |
| Sojalecithin (E322) | 50 mg |
| Hochdisperses Siliciumdioxid | 5 mg |

Kapselhülle:

| | |
|---|---|
| Gelatine | 303 mg |
| Glycerol 85 % | 87 mg |
| Sorbitol 70 % | 77 mg |
| gereinigtes Wasser | 52 mg |
| Eisenoxidpigment Braun 75 ( E 172) | 3 mg |

### b) Tablette mit Troxerutin und Aescin

(Troxerutin 250 mg + Aescin 13,75 mg)

| | |
|---|---|
| Troxerutin | 250 mg |
| Aescin | 13,75 mg |
| Milchzucker | 127,5 mg |
| Magnesiumstearat | 5 mg |
| Talcum | 23,75 mg |
| Mikrokristalline Cellulose | 81 mg |

### c) Hartgelatine-Kapsel mit Troxerutin und Aescin (Troxerutin 600 mg + Aescin 33 mg)

Füllung:

| | |
|---|---|
| Troxerutin | 600 mg |
| Aescin | 33 mg |
| Milchsäure | 272,5 mg |
| Magnesiumstearat | 14,5 mg |
| Talcum | 30 mg |
| Alginsäure | 50 mg |

d) Weiterhin können gemäß c) hergestellte Tabletten bzw. Dageekerne in bekannter Weise mit einem Magen- oder Dünndarm-löslichen Filmüberzug versehen werden.

### Beispiel 2

### Wirksamkeit

### Fallbeispiel 1

### 71-jähriger männlicher Patient mit Hörstörung.

Es bestand eine Innenohrschwerhörigkeit, die tonschwellenaudiometrisch insbesondere im Hochtonbereich nachgewiesen wurde. Der Befund bestand seit 8 Monaten mit der Tendenz zur Verschlechterung. Als Grundkrankheit lag ein generalisiertes sklerotisches Gefäßleiden vor; anamnestisch lag ferner ein mehrere Jahre zurückliegendes Knalltrauma vor.

Es erfolgte zunächst eine 4-wöchige Therapie mit 125 mg Aescin oral täglich. Hierunter besserte sich der Befund nicht. Tonschwellenaudiometrisch war keine Veränderung nachweisbar.

Es folgte nach einer dreiwöchigen Therapiepause eine 4-wöchige Therapie mit 2250 mg Troxerutin oral täglich. Hierunter kam es zu einer Verbesserung des Audiogramms um 10 dB des rechten Ohres im Hochtonbereich.

Nach einer 3-wöchigen Therapiepause kam es erneut zu einer Verschlechterung des Befundes. Es wurde die Therapie mit der Kombination von 2250 mg Troxerutin und 125 mg Aescin täglich oral durchgeführt. Nach 1 Woche kam es erneut zu einer Verbesserung des Audiogramms um 10 dB, nach 2 Wochen um 20 dB (gemessen vom Ausgangswert vor Therapiebeginn). Der Befund blieb dann bis zum Ende der Therapie konstant.

### Fallbeispiel 2

67-jährige weibliche Patientin mit langsam zunehmender Hörstörung, die seit 5 Monaten bestand.

Es bestand eine tonschwellenaudiometrisch nachweisbare Innenohrschwerhörigkeit, die sowohl im Tieftonbereich als auch im Hochtonbereich nachgewiesen wurde. Als Grundkrankheit lag neben einem sklerotischen Gefäßleiden auch ein latenter Diabetes mellitus vor.

Es erfolgte eine 4-wöchige Therapie mit 125 mg Aescin oral täglich. Der Befund besserte sich hierdurch nicht.

Es erfolgte nach einer 3-wöchigen Pause eine 3-wöchige Therapie mit 2250 mg Troxerutin oral täglich. Nach 1 Woche kam es zu einer Verbesserung des Audiogramms im Tieftonbereich um 10 dB, ferner zu einer geringgradigen Verbesserung im Hochtonbereich. Der weitere Verlauf war stationär.

Es folgte nach einer 3-wöchigen Therapiepause, in der es zu einer erneuten Verschlechterung im Tieftonbereich um 10 dB kann, eine 4-wöchige Therapie mit 2250 mg Troxerutin und 125 mg Aescin oral. Nach 1 Woche trat eine Verbesserung um 15 dB im Tiefton- und um 10 dB im Hochtonbereich und nach 3 Wochen eine Verbesserung um 25 dB im Tiefton- und um 20 dB im Hochtonbereich ein. Der Befund bleib dann bis zum Therapieende konstant.

### Fallbeispiel 3

32-jährige weibliche Patientin mit Tinnitus und Schwindelattacken seit 4 Monaten.

Es bestand ein tonschwellenaudiographisch nachweisbarer mittelgradiger Hörverlust insbesondere im Tieftonbereich und im Hochtonbereich. Als Grundkrankheit bestand ein insulinpflichtiger Diabetes mellitus. Der Befund bestand seit 4 Monaten mit der Tendenz zu zunehmender Verschlechterung.

Es folgte eine 4-wöchige Therapie mit 2.250 mg Troxerutin oral täglich. Hierunter kam es in der 1. Woche zu einer Verbesserung des Hörvermögens um 10 dB im Tieftonbereich und um 5 dB im Hochtonbereich. Die Schwindelattacken traten in unveränderter Häufigkeit (1- bis 4-mal innerhalb von 2 Tagen) auf. Der Tinnitus, der bisher ununterbrochen bestanden hatte, war in der 4. Behandlungswoche häufiger stundenweise unterbrochen.

Es schloss sich eine 3-wöchige Therapiepause an. Gegen Ende der Therapiepause verschlechterte sich das Hörvermögen erneut um 5 dB im Tieftonbereich und um 5 dB im Hochtonbereich. Der Tinnitus trat wieder unverändert auf.

Es begann nun die Therapie mit 125 mg Aescin oral täglich. Eine Besserung trat nicht auf, der Befund verschlechterte sich weiter um 5 dB im Tieftonbereich. Die Schwindelattacken traten unverändert auf, der Tinnitus bestand weiter wie zuvor.

Nach einer 3-wöchigen Therapiepause wurde die Therapie mit der Kombination von 2250 mg Troxerutin und 125 mg Aescin oral täglich über 3 Wochen durchgeführt. Nach einer Woche besserte sich das Hörvermögen um 15 dB im Tieftonbereich und um 10 dB im Hochtonbereich und nach drei Wochen um 25 dB im Tieftonbereich und um 15 dB im Hochtonbereich. Die Schwindelattacken verminderten sich in ihrer Häufigkeit (2 mal innerhalb einer Woche); in der 4. Behandlungswoche trat keine Schwindelattacke auf. Der Tinnitus war ab der 2. Woche stundenweise unterbrochen, in der 4. Woche war der Tinnitus etwa die Hälfte des Tages unterbrochen, an einem Tag war er vollständig unterbrochen.

### Fallbeispiel 4

36-jähriger männlicher Patent mit stationärer Hörstörung seit 4 Monaten.

Es bestand ein tonschwellenaudiometrisch nachweisbarer Hörsturz mit einem Hörverlust im Tieftonbereich und im Hochtonbereich. Anamnestisch lag ein Knalltrauma vor. Weitere Risikofaktoren bestanden nicht.

Es folgte eine 4-wöchige Therapie mit 125 mg Aescin täglich oral. Eine Befundänderung trat nicht ein.

Nach einer 3-wöchigen Therapiepause folgte eine Therapie mit 2250 mg Troxerutin täglich oral. In der 3. Woche besserte sich das Hörvermögen im Tieftonbereich um 10 dB und geringgradig im Hochtonbereich.

Es schloss sich eine 3-wöchige Therapiepause an, in deren Verlauf der Hörverlust im Tieftonbereich um 10 dB zunahm.

Es begann nun eine Therapie mit 2250 mg Troxerutin und 125 Aescin täglich oral. Nach 2 Wochen besserte sich das Hörvermögen im Tieftonbereich um 10 dB und im Hochtonbereich um 5 dB und nach 4 Wochen im Tieftonbereich um 20 dB und im Hochtonbereich um 10 dB.

## Patentansprüche

1. Verwendung wenigstens eines Rutins in Kombination mit wenigstens einem Aescin zur Herstellung pharmazeutischer Mittel zur Behandlung aurikulärer Durchblutungsstörungen.

2. Verwendung nach Anspruch 1, wobei das Rutin ein O-(β-Hydroxyethyl)rutin ist.

3. Verwendung nach Anspruch 2, wobei das O-(β-Hydroxyethyl)rutin Troxerutin ist.

4. Verwendung nach einem der vorhergehenden Ansprüche, wobei das Aescin in Form eines Roßkastaniensamenextrakts vorliegt.

5. Verwendung nach einem der Ansprüche 1 bis 4, wobei die Durchblutungsstörungen das Innenohrs oder die Hörbahn betreffen.

6. Verwendung nach Anspruch 5, wobei die Durchblutungsstörungen Mikroperfusionstörungen des Innenohrs oder des zentralen Hörzentrums im Gehirn sind.

7. Verwendung nach Anspruch 6, wobei die Mikroperfusionstörungen die Stria vascularis, die Cochlea, den Hörnerv, das Ganglion spirale, die Hörbahn, oder den Temporallappen des Gehirns betreffen.

8. Verwendung nach einem der Ansprüche 1 bis 4, wobei die Durchblutungsstörungen Schwerhörigkeit, Gleichgewichtsstörungen und/oder Schwindel, Ohrgeräusche oder vasculär bedingter Hörsturz sind.

9. Verwendung nach Anspruch 8, wobei die Schwerhörigkeit Altersschwerhörigkeit ist.

10. Verwendung nach einem der vorhergehenden Ansprüche, wobei man einem Individuum eine Tagesdosis von mehr als 1800 mg wenigstens eines Rutins und von etwa 1 mg bis 500 mg wenigstens Aescins oral bzw. bioäquivalente Mengen davon auf anderem Wege verabreicht.

## Claims

1. The use of at least one rutin in combination with at least one escin for the production of pharmaceutical compositions for the treatment of circulatory disturbances of the ear.

2. The use as claimed in claim 1, wherein the rutin is an O-(β-hydroxyethyl)rutin.

3. The use as claimed in claim 2, wherein the O-(β*-hydroxyethyl)rutin is troxerutin.

4. The use as claimed in one of the preceding claims, wherein the escin is present in the form of a horse chestnut seed extract.

5. The use as claimed in one of claims 1 to 4, wherein the circulatory disturbances affect the inner ear or the auditory pathway.

6. The use as claimed in claim 5, wherein the circulatory disturbances are microcirculatory disturbances of the inner ear or the auditory center in the brain.

7. The use as claimed in claim 6, wherein the microcirculatory disturbances affect the vascular stria, the cochlea, the auditory nerve, the spiral ganglion, the auditory pathway or the temporal lobe of the brain.

8. The use as claimed in one of claims 1 to 4, wherein the circulatory disturbances are hearing impairment, disturbances of balance and/or vertigo, noises in the ear or sudden deafness of vascular origin.

9. The use as claimed in claim 8, wherein the hearing impairment is presbyacusis.

10. The use as claimed in one of the preceding claims, where a daily dose of more than 1800 mg of at least one rutin and of approximately 1 mg to 500 mg of at least one escin are administered orally to an individual or bioequivalent amounts thereof are administered in another way.

## Revendications

1. Utilisation d'au moins une rutine en combinaison avec au moins une aescine pour préparer un produit pharmaceutique destiné au traitement des troubles de la circulation sanguine des oreilles.

2. Utilisation selon la revendication 1, dans laquelle la rutine est une O-(β-hydroxyéthyl)rutine.

3. Utilisation selon la revendication 2, dans laquelle la O-(β-hydroxyéthyl)rutine est la troxérutine.

4. Utilisation selon l'une quelconque des revendications précédentes, dans laquelle l'aescine se présente sous la forme d'un extrait de marron d'Inde.

5. Utilisation selon l'une quelconque des revendications 1 à 4, dans laquelle les troubles de la circulation sanguine concernent l'oreille interne ou la voie acoustique.

6. Utilisation selon la revendication 5, dans laquelle les troubles de la circulation sanguine sont des troubles de microperfusion de l'oreille interne ou du centre auditif central dans le cerveau.

7. Utilisation selon la revendication 6, dans laquelle les troubles de microperfusion concernent la strie vasculaire, la cochlée, le nerf auditif, le ganglion spiralé, la voie acoustique ou le lobe temporal du cerveau.

8. Utilisation selon les revendications 1 à 4, dans laquelle les troubles de la circulation sanguine sont l'hypoacousie, les troubles de l'équilibre et/ou le vertige, les acouphènes ou la perte brusque de l'audition pour des raisons vasculaires.

9. Utilisation selon la revendication 8, dans laquelle l'hypoacousie est une presbyacousie.

10. Utilisation selon l'une quelconque des revendications précédentes, dans laquelle on administre à un individu une dose quotidienne supérieure à 1800 mg d'au moins une rutine et d'environ 1 à 500 mg au moins d'aescine par voie orale ou des quantités bioéquivalentes de celles-ci administrées différemment.
